# EUROPEAN PATENT SPECIFICATION

(11) **EP 2 343 055 B1**
(45) Date of publication and mention of the grant of the patent: **18.10.2017**
(21) Application number: 10196217.3
(22) Date of filing: 21.12.2010
(51) Int. Cl.: A61K 9/14, A61K 9/20, A61K 9/48, A61K 31/197

(54) **Pharmaceutical compositions of pregabalin**
Pharmazeutische Zusammensetzungen mit Pregabalin
Compositions pharmaceutiques de prégabaline

(30) Priority: 22.12.2009 TR 200909613
(43) Date of publication of application: 13.07.2011
(73) Proprietor: Arven Ilac Sanayi ve Ticaret A.S., Istinye, Istanbul 34460 (TR)
(72) Inventor: Toksöz, Ahmet, 34398, Istanbul (TR); Türkyilmaz, Ali, 34398, Istanbul (TR); Akalin, Nur Pehlivan, 34398, Istanbul (TR); Yildirim, Aylin, 34398, Istanbul (TR); Demir, Vildan, 34398, Istanbul (TR); Öner, Levent, 34398, Istanbul (TR)
(74) Representative: Sevinç, Erkan

(56) References cited:
- WO-A1-2009/066325
- WO-A2-2008/128775
- US-A1- 2002 012 679
- US-A1- 2002 098 227

## Description

### Field of Invention

The present invention relates to pharmaceutical compositions of pregabalin or a pharmaceutically acceptable salt thereof. The present invention more particularly relates to a stable capsule form of pregabalin, which is stable against ambient influences.

### Background of Invention

Pregabalin is an analog of gamma-aminobutyric acid (GABA). Its chemical designation is (S)-3-(aminomethyl)-5-methyl hexanoic acid, with the chemical structure illustrated below with Formula 1.

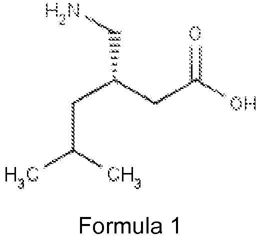

It is known that pregabalin binds to the auxiliary subunit of voltage-sensitive calcium channels in the central nervous system, thereby replacing the [3H]-gabapentin. It also reduces the release of many neurotransmitters, including pregabalin glutamate, noradrenaline and the substance P. It is used for the treatment of epilepsy, simple or complex partial convulsion, either accompanied or not by secondary generalized convulsions, and of neuropathic pain.

There have been various patents available in the patent literature.

The patent application WO2008008120, for instance, discloses a solid dosage form comprising a compacted fill material containing at least one active agent and at least one of a disintegrating agent and wetting agent.

The patent application WO2008140459, in turn, discloses a solid dosage form comprising a compacted fill material containing a pressure-sensitive multi-particulate and at least one cushioning agent. Said multi-particulate and/or cushioning agent comprises at least one active agent and display(s) a weight loss less than 1% in 30 minutes according to the friability test USP 29 test number 1216. The compacted fill material has a density of at least 0.5 g/ml and a tensile strength of less than 0.9 MPa.

The patent application WO2008140460, on the other hand, claims a solid dosage form comprising a compacted fill material including at least one active agent. The solid dosage form displays a weight loss of less than 1% in 30 minutes in accordance with the friability test USP 29 test number 1216. The compacted fill material particles contain at least one active agent in the matrix and provides a controlled release of the active agent.

The patent application WO2008128775 claims a solid pharmaceutical composition comprising pregabalin as an active agent, together with one or more auxiliary agents. Said composition is free from saccharides and comprises no further amino acids.

In the aforementioned applications, said compositions are compressed and are coated with a water-soluble film. Any stability problem to occur due to storage and ambient conditions are eliminated in this manner according to said applications.

Forming the tablet by means of compacting and coating it with an air-impermeable film-like barrier enhance the stability, but such an approach increases the number of process steps.

Stability-related problems do occur in a plurality of active agents, including pregabalin, with the influence of ambient and physical conditions. Many patent applications have been made, including those referred to above, for eliminating such kind of problems and for enhancing the stability.

One of problems encountered during the production of medicaments in the dosage forms of powder, granule, tablet, or capsule is the lowering of flowability during the filling step and the aggregation of materials being filled.

Whilst granule- or particle-coating processes are made, these processes make use of organic solvents and polymeric substances. Such coating processes, however, do accelerate the dissolution rate, thereby directly effecting the bioavailability of drug. Subjecting active agents to processes involving solvents or water, on the other hand, influences the stability negatively.

In result, the aforesaid drawbacks require a novelty in the art of pregabalin formulations with antiepileptic, analgesic, and anxiolytic activities.

### Object and Brief Description of Invention

The present invention relates to a pregabalin formulation, eliminating all aforesaid problems and bringing additional advantages to the relevant prior art.

Accordingly, the main object of the present invention is to obtain a stable formulation with antiepileptic, analgesic, and anxiolytic activities.

Another object of the present invention is to ensure the stabilization of pregabalin particles or granules used in said formulation without the dissolution rate and bioavailability of the same being effected.

A further object of the present invention is to protect the pregabalin particles or granules used in said formulation against external effects by means of dry-coating with colloidal silicon dioxide without requiring any chemical process.

Yet a further object of the present invention is to avoid aggregation by dry-coating the pregabalin particles or granules used in said formulation with colloidal silicon dioxide without requiring any chemical process, and to ensure flowability at desired extent during the filling process.

A solid pharmaceutical formulation is realized to carry out all objects, referred to above and to emerge from the following detailed description.

The present invention is defined in claims 1-7.

According to a preferred embodiment of the present invention, said novelty is carried out with pregabalin or a pharmaceutically acceptable salt thereof and colloidal silicon dioxide. The surface of pregabalin particles or granules is dry-coated with colloidal silicon dioxide.

According to a preferred embodiment of the present invention, said formulation is in the form of capsules, tablets, powders, granules, or sachets. Said formulation is preferably a capsule.

Another preferred embodiment according to the present invention further comprises at least one or a proper mixture of microcrystalline cellulose, dicalcium phosphate, talc, calcium carbonate, dibasic calcium phosphate, dextrin, dextrose, ethyl cellulose, fructose, lactitol, starch, pregelatinized starch, corn starch, sucrose, xylitol, maltodextrin, magnesium carbonate, maltose, mannitol, polydextrose, sodium alginate, sodium chloride, sorbitol, carbomer, hydroxypropyl methyl cellulose, sodium carboxymethyl cellulose, gelatin, hydroxyethyl cellulose, hydroxypropyl cellulose, methylcellulose, povidone, sodium bicarbonate, alginic acid, calcium carboxymethyl cellulose, magnesium stearate, calcium stearate, croscarmellose sodium, sodium starch glycolate, crospovidone, sodium lauryl sulfate, colloidal silicon dioxide. Preferably dibasic calcium phosphate and corn starch are included into the formulation according to the present invention.

A further embodiment of the present invention provides a method for preparing the pharmaceutical formulation according to the present invention, this method comprising the steps of
a. mixing together pregabalin and silicon dioxide in order to provide the dry-coating of particles or granules of pregabalin or a pharmaceutically acceptable salt thereof with colloidal silicon dioxide,
b. then adding dibasic calcium phosphate and corn starch into this mixture and performing a second mixing step, and
c. filling the capsules or compressing the tablets.

In another preferred embodiment of the present invention, said pharmaceutical formulation contains the following ingredients only:
a. pregabalin or a pharmaceutically acceptable salt thereof at 25-90% by weight
b. colloidal silicon dioxide at 0.1-5% by weight
c. dibasic calcium phosphate at 1-70% by weight
d. corn starch at 1-70% by weight.

### Detailed Description of Invention

### Example 1:

| Content | Amount (mg) |
|---|---|
| Pregabalin | 300.0 |
| Colloidal silicon dioxide | 3.0 |
| Corn starch | 20.0 |
| Dibasic calcium phosphate | 77.0 |

Pregabalin and silicon dioxide are mixed together in order to provide the dry-coating of particles or granules of pregabalin or a pharmaceutically acceptable salt thereof with colloidal silicon dioxide. Dibasic calcium phosphate and corn starch are then added into the so-obtained mixture and a second mixing operation is performed. Calcium phosphate may be preferred from at least one or a mixture of dibasic calcium phosphate anhydrate, dibasic calcium phosphate dihydrate, tribasic calcium phosphate. The mixture formed as described above is filled into capsules or compressed in the form of tablets.

This formulation embodiment has surprisingly resulted in a quite satisfactory dissolution rate, without causing any aggregation and flowability problem in said particles or granules during the filling operation.

Mixing pregabalin particularly with colloidal silicon dioxide results in the dry-coating of pregabalin particles or granules with colloidal silicon dioxide. The term dry-coating here refers to coating the pregabalin particles or granules by making use of the adsorbent character of silicon dioxide. Pregabalin is mixed with silicon dioxide for this purpose during the first step of process. Thanks to this operation, ambient effects such as heat, air, humidity on pregabalin particles or granules are minimized. This operation enhances the stability of the active agent on one hand, and on the other hand, it does not cause any drawback on the dissolution rate of the active agent due to the high solubility and dissolution rate of silicon dioxide. This operation also avoids aggregation and other factors negatively influencing the flowability.

Stability and dissolution rate results for 300 mg capsule formulation as follows

| | |
|---|---|
| **300 mg Capsule** | **Serial Number: 01109** |

| **TESTS** | **Specification** | **starting** | **3 rd month** 40 °C %75RH | **6 th month** 40 °C %75RH |
|---|---|---|---|---|
| **Dissolution rate (Pregabalin) 15 th minutes result** | The dissolution rate study was investigated 900 mL, 0.06 N HCl, 50 rpm | %97 | %99 | %97 |
| **impurity** (**Ph**. **Eur.)** | | % 0.00 | % 0.00 | % 0.01 |
| | | % 0.03 | % 0.03 | % 0.03 |
| | | % 0.07 | % 0.08 | % 0.08 |
| **Active ingredient (Ph. Eur.) (300.0 mg Pregabalin**) | 300.0 mg ± %10.0 / Capsule (330.0 - 270.0 mg / Capsule) | 302.2mg / capsule | 302.6 mg/capsule | 302.1 mg/capsule |

| | |
|---|---|
| **300 mg Capsule** | **Serial number: 02109** |

| **TESTS** | **Specification** | **Starting** | **3 rd month** 40 °C %75RH | **6 th month** 40 °C %75RH |
|---|---|---|---|---|
| **Dissolution rate (Pregabalin) 15 th minutes result** | The dissolution rate study was investigated 900 mL, 0.06 N HCl, 50 rpm | % 96 | % 99 | % 97 |
| **impurity** (**Ph**. **Eur.)** | | % 0.00 | % 0.00 | % 0.01 |
| | | % 0.03 | % 0.02 | % 0.02 |
| | | % 0.08 | % 0.07 | % 0.08 |
| **Active ingredient ( Ph. Eur.) (300.0 mg Pregabalin**) | 300.0 mg ± %10.0 / Capsule (330.0 - 270.0 mg / Capsule) | 298.4mg / capsule | 304.1 mg/capsule | 301.5 mg/capsule |

The pharmaceutical compositions according to the present invention may also comprise one or more pharmaceutically acceptable excipients. Such pharmaceutically acceptable excipients include, but are not restricted to fillers, glidants, lubricants, disintegrants, surface active agents etc. and the mixtures thereof.

The present invention is used for the treatment of epilepsy, pain, anxiety, diabetic neuropathy, neuropathic pain, postherpetic neuralgia.

The present invention is hereby disclosed by referring to an exemplary embodiment hereinabove. Whilst this exemplary embodiment does not restrict the object of the present invention, the latter must be assessed under the light of the foregoing detailed description.

## Claims

1. A solid pharmaceutical formulation comprising pregabalin or a pharmaceutically acceptable salt thereof, **characterized in that** particles or granules of pregabalin or a pharmaceutically acceptable salt thereof are dry-coated with colloidal silicon dioxide, and the formulation further comprises dibasic calcium phosphate and corn starch.

2. The pharmaceutical formulation according to any of the preceding claims, this formulation being in the form of capsules, tablets, powders, granules, or sachets.

3. The pharmaceutical formulation according to claims 1 and 2, this formulation being preferably in the form of capsules.

4. The pharmaceutical formulation according to any of the preceding claims, further comprising at least one or a proper mixture of microcrystalline cellulose, talc, dicalcium phosphate, lactose, calcium carbonate, dibasic calcium phosphate, dextrin, dextrose, ethyl cellulose, fructose, lactitol, starch, pregelatinized starch, corn starch, sucrose, xylitol, maltodextrin, magnesium carbonate, maltose, mannitol, polydextrose, sodium alginate, sodium chloride, sorbitol, carbomer, hydroxypropyl methyl cellulose, sodium carboxymethyl cellulose, gelatin, hydroxyethyl cellulose, hydroxypropyl cellulose, methylcellulose, povidone, sodium bicarbonate, alginic acid, calcium carboxymethyl cellulose, magnesium stearate, calcium stearate, croscarmellose sodium, sodium starch glycolate, crospovidone, sodium lauryl sulfate, colloidal, silicon dioxide.

5. A method for preparing a pharmaceutical formulation, **characterized by** comprising the steps of:
a. mixing together pregabalin and silicon dioxide in order to provide the dry-coating of particles or granules of pregabalin or a pharmaceutically acceptable salt thereof with colloidal silicon dioxide,
b. then adding dibasic calcium phosphate and corn starch into this mixture and performing a second mixing step, and
c. filling the capsules or compressing the tablets.

6. A pharmaceutical formulation according to any of the preceding claims, consisting of
a. pregabalin or a pharmaceutically acceptable salt thereof at 25-90% by weight
b. colloidal silicone dioxide at 0.1-5% by weight
c. dibasic calcium phosphate at 1-70% by weight
d. corn starch at 1-70% by weight.

7. Use of a pharmaceutical formulation according to any of the preceding claims for the manufacture of a medicament for the treatment of epilepsy, pain, anxiety, diabetic neuropathy, neuropathic pain, postherpetic neuralgia.

## Patentansprüche

1. Feste pharmazeutische Formulierung, die Pregabalin oder ein pharmazeutisch verträgliches Salz davon umfasst, **dadurch gekennzeichnet, dass** Partikel oder Granulat von Pregabalin oder einem pharmazeutisch verträglichen Salz davon mit kolloidalem Siliciumdioxid trockenbeschichtet sind und die Formulierung ferner dibasisches Calciumphosphat und Speisestärke umfasst.

2. Pharmazeutische Formulierung nach einem der vorhergehenden Ansprüche, wobei diese Formulierung in Form von Kapseln, Tabletten, Pulvern, Granulaten oder Beuteln vorliegt.

3. Pharmazeutische Formulierung nach Anspruch 1 und 2, wobei diese Formulierung vorzugsweise in Form von Kapseln vorliegt.

4. Pharmazeutische Formulierung nach einem der vorhergehenden Ansprüche, ferner umfassend wenigstens eines aus oder ein zweckmäßiges Gemisch aus mikrokristalliner Cellulose, Talkum, Dicalciumphosphat, Lactose, Calciumcarbonat, dibasischem Calciumphosphat, Dextrin, Dextrose, Ethylcellulose, Fructose, Lactitol, Stärke, Quellstärke, Speisestärke, Saccharose, Xylit, Maltodextrin, Magnesiumcarbonat, Maltose, Mannit, Polydextrose, Natriumalginat, Natriumchlorid, Sorbit, Carbomer, Hydroxypropylmethylcellulose, Natriumcarboxymethylcellulose, Gelatine, Hydroxyethylcellulose, Hydroxypropylcellulose, Methylcellulose, Povidon, Natriumbicarbonat, Alginsäure, Calciumcarboxymethylcellulose, Magnesiumstearat, Calciumstearat, Croscarmellose-Natrium, Natriumstärkeglykolat, Crospovidon, Natriumlaurylsulfat, kolloidalem Siliciumdioxid.

5. Verfahren zur Herstellung einer pharmazeutischen Formulierung, **dadurch gekennzeichnet, dass** es die folgenden Schritte umfasst:
a. Zusammenmischen von Pregabalin und Siliciumdioxid, um die Trockenbeschichtung von Partikeln oder Granulat von Pregabalin oder einem pharmazeutisch verträglichen Salz davon mit kolloidalem Siliciumdioxid zu gewährleisten,
b. anschließende Zugabe von dibasischem Calciumphosphat und Speisestärke zu dieser Mischung und Durchführen eines zweiten Mischschritts, und
c. Befüllen der Kapseln oder Verpressen zu Tabletten.

6. Pharmazeutische Formulierung nach einem der vorhergehenden Ansprüche, bestehend aus
a. Pregabalin oder einem pharmazeutisch verträglichen Salz davon zu 25-90 Gew.%
b. kolloidalem Siliciumdioxid zu 0,1-5 Gew.-%
c. dibasischem Calciumphosphat zu 1-70 Gew.-%
d. Speisestärke zu 1-70 Gew.-%.

7. Verwendung einer pharmazeutischen Formulierung nach einem der vorhergehenden Ansprüche zur Herstellung eines Medikaments zur Behandlung von Epilepsie, Schmerzen, Angst, diabetischer Neuropathie, neuropathischen Schmerzen, postherpetischer Neuralgie.

## Revendications

1. - Formulation pharmaceutique solide comprenant de la prégabaline ou un sel pharmaceutiquement acceptable de celle-ci, **caractérisée par le fait que** des particules ou des granulés de prégabaline ou d'un sel pharmaceutiquement acceptable de celle-ci sont enrobés à sec par du dioxyde de silicium colloïdal, et la formulation comprend en outre du phosphate de calcium dibasique et de l'amidon de maïs.

2. - Formulation pharmaceutique selon l'une quelconque des revendications précédentes, cette formulation se présentant sous la forme de gélules, de comprimés, de poudres, de granulés ou de sachets.

3. - Formulation pharmaceutique selon l'une des revendications 1 et 2, cette formulation se présentant de préférence sous la forme de gélules.

4. - Formulation pharmaceutique selon l'une quelconque des revendications précédentes, comprenant en outre au moins un ou un mélange approprié de cellulose microcristalline, talc, phosphate dicalcique, lactose, carbonate de calcium, phosphate de calcium dibasique, dextrine, dextrose, éthyl cellulose, fructose, lactitol, amidon, amidon prégélatinisé, amidon de maïs, sucrose, xylitol, maltodextrine, carbonate de magnésium, maltose, mannitol, polydextrose, alginate de sodium, chlorure de sodium, sorbitol, carbomère, hydroxypropyl méthyl cellulose, carboxyméthyl cellulose sodique, gélatine, hydroxyéthyl cellulose, hydroxypropyl cellulose, méthylcellulose, povidone, bicarbonate de sodium, acide alginique, carboxyméthyl cellulose calcique, stéarate de magnésium, stéarate de calcium, croscarmellose sodique, glycolate d'amidon sodique, crospovidone, lauryl sulfate de sodium, dioxyde de silicium colloïdal.

5. - Procédé de préparation d'une formulation pharmaceutique, **caractérisé par le fait qu'**il comprend les étapes de :
a. mélanger ensemble de la prégabaline et du dioxyde de silicium de façon à fournir l'enrobage à sec de particules ou de granulés de prégabaline ou d'un sel pharmaceutiquement acceptable de celle-ci par du dioxyde de silicium colloïdal ;
b. puis ajouter du phosphate de calcium dibasique et de l'amidon de maïs dans ce mélange et effectuer une seconde étape de mélange ; et
c. remplir les gélules ou compresser les comprimés.

6. - Formulation pharmaceutique selon l'une quelconque des revendications précédentes, consistant en :
a. prégabaline ou sel pharmaceutiquement acceptable de celle-ci à raison de 25-90 % en poids ;
b. dioxyde de silicium colloïdal à raison de 0,1-5 % en poids ;
c. phosphate de calcium dibasique à raison de 1-70 % en poids ;
d. amidon de maïs à raison de 1-70 % en poids.

7. - Utilisation d'une formulation pharmaceutique selon l'une quelconque des revendications précédentes pour la fabrication d'un médicament pour le traitement de l'épilepsie, de la douleur, de l'anxiété, de la neuropathie diabétique, de la douleur neuropathique, de la névralgie post-herpétique.
